# EUROPEAN PATENT APPLICATION

(11) **EP 3 984 547 A1**
(43) Date of publication of application: **20.04.2022**
(21) Application number: 20382901.5
(22) Date of filing: 14.10.2020
(51) Int. Cl.: A61K 38/08, A61K 38/10, A61P 35/00

(54) **PEPTIDES FOR THE TREATMENT OF CANCER**

(71) Applicant: Universitat Pompeu Fabra, 08002 Barcelona (ES); Fundació Clinic per a la Recerca Biomèdica (FCRB), 08036 Barcelona (ES); Fundacio Universitaria Balmes, 08500 Vic (ES)
(72) Inventor: OLIVA MIGUEL, Baldomero, E-08003 Barcelona (ES); ANDREU MARTÍNEZ, David, E-08003 Barcelona (ES); SIERRA JIMÉNEZ, Àngels, E- 08208 Sabadell (ES); FERNANDEZ FUENTES, Narcís, E-08500 Vic - Barcelona (ES)
(74) Representative: ABG Intellectual Property Law, S.L.

(57) **Abstract**

The present invention relates to peptides, compositions comprising said peptides as well as the use of said peptides in the treatment and/or prevention of Fn-14 positive solid cancers, such as brain cancer, and cancer-induced cachexia.

SEQUENCE LISTING

<110> UNIVERSITAT POMPEU FABRA
FUNDACIÓ CLÍNIC PER LA RECERCA BIOMÈDICA
FUNDACIÓ UNIVERSITÀRIA BALMES

<120> PEPTIDES FOR THE TREATMENT OF CANCER

<130> P19737EP00

<160> 4

<170> PatentIn version 3.5

<210> 1
<211> 12
<212> PRT
<213> Artificial Sequence

<220>
<223> synthetic peptide

<400> 1

Thr Thr Asp Gly Ser Thr Gly Asp Asp Leu Gln Ala
1               5                   10

<210> 2
<211> 12
<212> PRT
<213> Artificial Sequence

<220>
<223> synthetic peptide

<400> 2

Ser Trp Tyr Asp His Met Ala Gly Asp Ser Ser Tyr
1               5                   10

<210> 3
<211> 8
<212> PRT
<213> Artificial Sequence

<220>
<223> synthetic peptide

<400> 3

Ser Tyr Thr Asp Val Gln Asp Ser
1               5

<210> 4
<211> 12
<212> PRT
<213> Artificial Sequence

<220>
<223> synthetic peptide

<400>  4

Asp Asn Gly Gly Gly Asn Thr Asp Ile Tyr Ser Glu
1               5                   10

## Description

### FIELD OF THE INVENTION

The invention relates to peptides and compositions which are useful for the treatment and/or prevention of an Fn-14 positive solid cancer, in particular brain cancer, as well as cancer-induced cachexia.

### BACKGROUND OF THE INVENTION

Cancer is a large group of diseases that can start in almost any organ or tissue of the body when abnormal cells grow uncontrollably, go beyond their usual boundaries to invade adjoining parts of the body and/or spread to other organs. The latter process is called metastasizing and is a major cause of death from cancer.

According to the World Health Organization (WHO), cancer is the second leading cause of death globally, accounting for an estimated 9.6 million deaths, or one in six deaths, in 2018. Lung, prostate, colorectal, stomach and liver cancer are the most common types of cancer in men, while breast, colorectal, lung, cervical and thyroid cancer are the most common among women. All of them being solid cancers.

Main treatment options for solid cancers include surgery, chemotherapy, radiation therapy, hormonal therapy, targeted therapy and palliative care. The efficacy of chemotherapy depends on the type of cancer and the stage. In combination with surgery, chemotherapy has proven useful in a number of different solid cancer. The effectiveness of chemotherapy is often limited by toxicity to other tissues in the body. Even when it is impossible for chemotherapy to provide a permanent cure, chemotherapy may be useful to reduce symptoms like pain or to reduce the size of an inoperable tumor in the hope that surgery will be possible in the future.

The tumor necrosis factor-like weak inducer of apoptosis (TWEAK) is a typical member of the TNF ligand family, constitutively expressed in monocytes and some tumor cell types and initially shown to induce apoptosis of malignant cells (reviewed in Hu et al., Tumor Biology, 2017, 39, 1-9). The only TWEAK receptor reported so far is fibroblast growth factor-inducible 14 (Fn14), a type I transmembrane protein [Chicheportiche et al., J Biol Chem., 1997, 272, 32401-32410; Wiley et al., Immunity, 2001, 15, 837-846]. Fn14 contains the extracellular domain that binds to TWEAK as well as the cytoplasmic tail region essential for signal transduction [Winkles, Nat Rev Drug Discov., 2008, 7, 411-425]. Both TWEAK and Fn14 show low expression in normal tissues but are highly expressed in many tumors and metastases, associated with a worse clinical outcome. TWEAK is particularly overexpressed in solid tumors [Culp et al., Clin Cancer Res., 2010, 16, 497-508], in particular in liver, colorectal, esophageal, bladder, pancreatic, ovarian and prostate cancers as well as neuroblastoma [Hu et al., Tumor Biology, 2017, 39, 1-9]. Besides, an increase expression of Fn14 has been reported in brain glioma [Tan et al., Future Oncol., 2018, 14(13), 1273-1284], breast cancer, lung cancer [Wang et al., Med Sci Monit., 2018, 24, 1282-1294; Whitsett et al., Mol Cancer Res., 2014, 12(4), 550-559], and melanoma [Hu et al., Tumor Biology, 2017, 39, 1-9]. Additional, Fn14 is highly expressed in the metastases of breast, colorectal, melanoma, non-small cell lung, and prostate cancers [Hu et al., Tumor Biology, 2017, 39, 1-9]. The pro-tumoral role of TWEAK-Fn14 in cancer depends on the nature and degree of Fn14 engagement [Burkly, Semin Immunol., 2014, 26, 229-236]. TWEAK-Fn14 triggers intracellular signaling cascades through activating the TRAF2 and NF-κB pathways that lead to downstream cellular responses associated with cell invasion and migration, as well as proliferation and angiogenesis [Hu et al., Tumor Biology, 2017, 39, 1-9; Wajant, Br J Pharmacol., 2013, 170, 748-764].

In the central nervous system (CNS), TWEAK targets endothelial cells, astrocytes and neurons, inducing an inflammatory profile [Rousselet et al., Mol Pharmacol., 2012, 82, 948-957; Stephan et al., J Neuroinflammation, 2013, 10, 9, doi:10.1186/1742-2094-10-9] that causes disruption of the blood brain barrier (BBB) by way of proinflammatory cytokines [Boulamery and Desplat-Jego, Front Immunol., 2017, 8, 1534]. Since BBB is critical to effective delivery therapies, the TWEAK-Fn14 axis is involved in tumor resistance, mainly by inducing the transition of tumor cells from an invasive to a proliferative state, with mitogenic activity mediated by the TWEAK-induced MAPK signaling pathway [Vicent et al., J Bone Miner Res., 2009, 24, 1434-1449]. TWEAK has been found overexpressed in the invasive front of brain metastases [Martinez-Aranda et al., Oncotarget, 2015, 6, 44254-44273]. For its part, Fn14 gene expression is low in normal brain tissue but upregulated in advanced brain cancers, with maximal levels in the invading cells within normal brain tissue [Perez et al., Oncogene, 2016, 35, 2145-2155].

In this context, specific cancer therapies targeting the TWEAK-Fn14 have been proposed [Martinez-Aranda et al., Front Oncol., 2017, 7, 283; Meulendijks et al., Clin Cancer Res., 2016, 22, 858-867; Lassen et al., Clin Cancer Res., 2015, 21, 258-266].

It is estimated that half of all patients with cancer eventually develop a syndrome of cachexia, with anorexia and a progressive loss of adipose tissue and skeletal muscle mass. Cancer cachexia is characterized by systemic inflammation, negative protein and energy balance, and an involuntary loss of lean body mass. It is an insidious syndrome that not only has a dramatic impact on patient quality of life, but also is associated with poor responses to chemotherapy and decreased survival. TWEAK-Fn14 signaling has also been reported to play a role in muscle wasting and cachexia [Ebner and Heahling, Journal of Cachexia, Sarcopenia and Muscle, 2016, 7, 90-94; and Johnston et al., Cell, 2015, 162(6), 1365-1378].

In view of the above, there is a need for new therapies that target the TWEAK-Fn14 interaction and which are thus useful for the treatment and prevention of Fn-14 positive solid tumors, in particular of brain tumors, including brain metastases, as well as cancer-induced cachexia.

### BRIEF DESCRIPTION OF THE INVENTION

The inventors have found four new peptides that are capable of modulating the TWEAK-Fn14 interaction, as shown in the examples of the present invention.

Thus, in a first aspect, the present invention is directed to a peptide selected from the group consisting of TTDGSTGDDLQA (SEQ ID NO: 1), SWYDHMAGDSSY (SEQ ID NO: 2), SYTDVQDS (SEQ ID NO: 3) and DNGGGNTDIYSE (SEQ ID NO: 4) or a pharmaceutically acceptable salt thereof.

In a second aspect, the present invention relates to a composition comprising a peptide according to the first aspect and a pharmaceutically acceptable excipient.

In a third aspect, the present invention relates to a peptide according to the first aspect or a composition according to the second aspect, for use in medicine.

In a fourth aspect, the present invention relates to a peptide according to the first aspect or a composition according to the second aspect, for use in the treatment and/or prevention of a Fn-14 positive solid cancer, in particular a brain cancer.

In a fifth aspect, the present invention relates to a peptide according to the first aspect or a composition according to the second aspect, for use in the treatment and/or prevention of cancer-induced cachexia.

### DESCRIPTION OF THE FIGURES

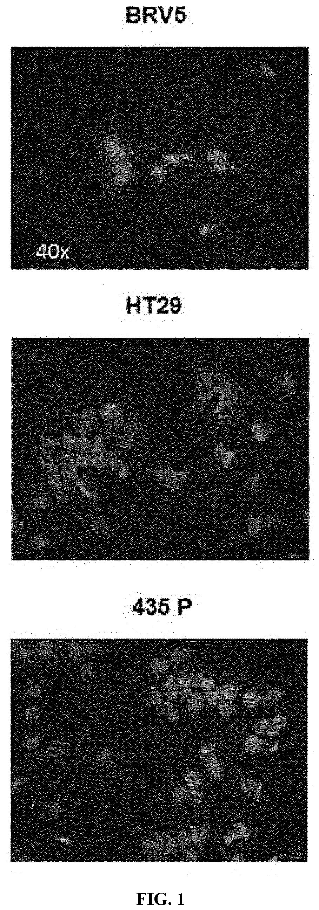

Figure 1. Representative images of Fn14 expression in BRV5, 435-P and HT29 cells labelled by immunofluorescence. The protein is mainly located in cellular membrane and cytoplasm, with similar expression in BRV5 and the positive control HT29 cells, and less expressed in 435-P. DAPI was used for nucleus identification. Scale bars 50 µm.

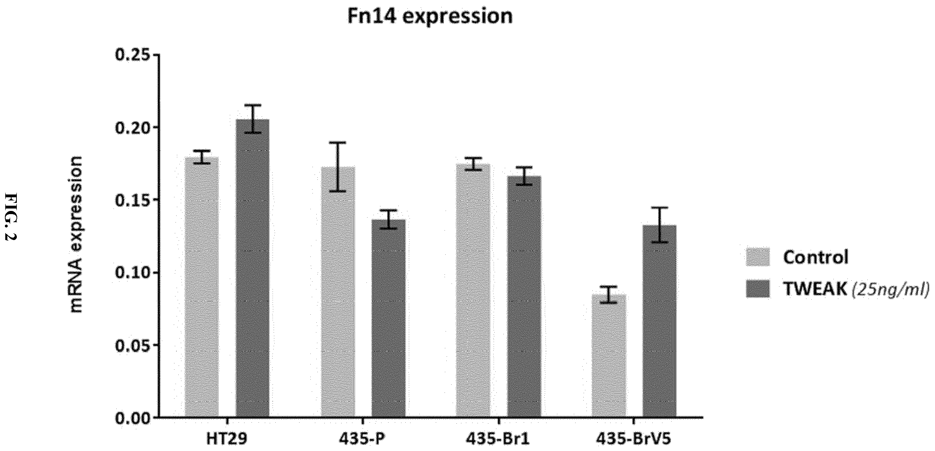

Figure 2. RT-PCR analysis to quantify Fn14 gene expression of cells cultured in the presence/or not of TWEAK (25 ng/ml) 48 h. Upregulation of Fn14 is observed in HT29 and BRV5 cells. Error bars represent the standard deviation (s.d.) of technical replicates (one representative of 3 experiments).

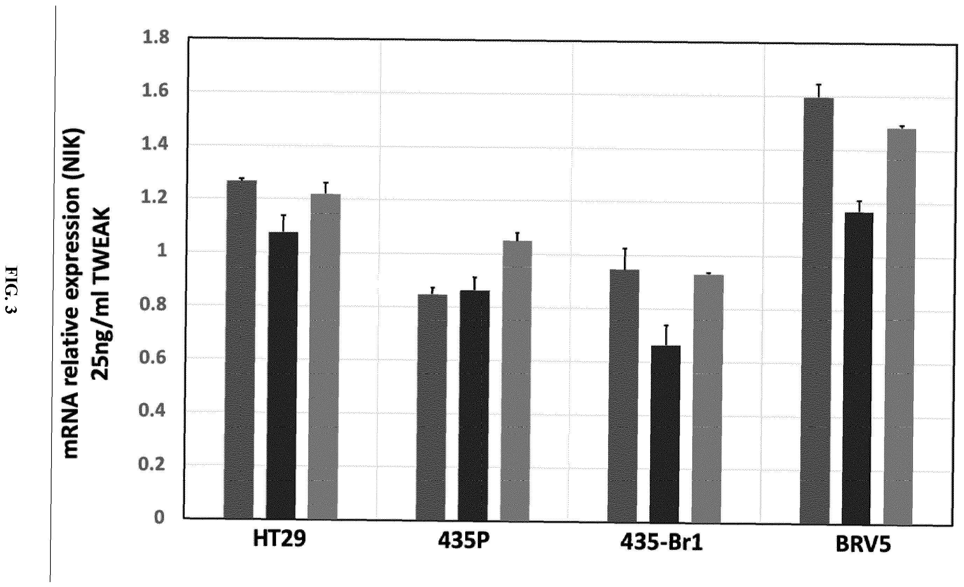

Figure 3. NIK activation in HT29 and BRV5 cells. Cells were primed with TWEAK in a time-course RT-PCR experiment. The bar chart shows activation of NIK (shown as mRNA relative expression) at 6 hours (left bar), overnight (middle bar) and 24 hours (right bar).

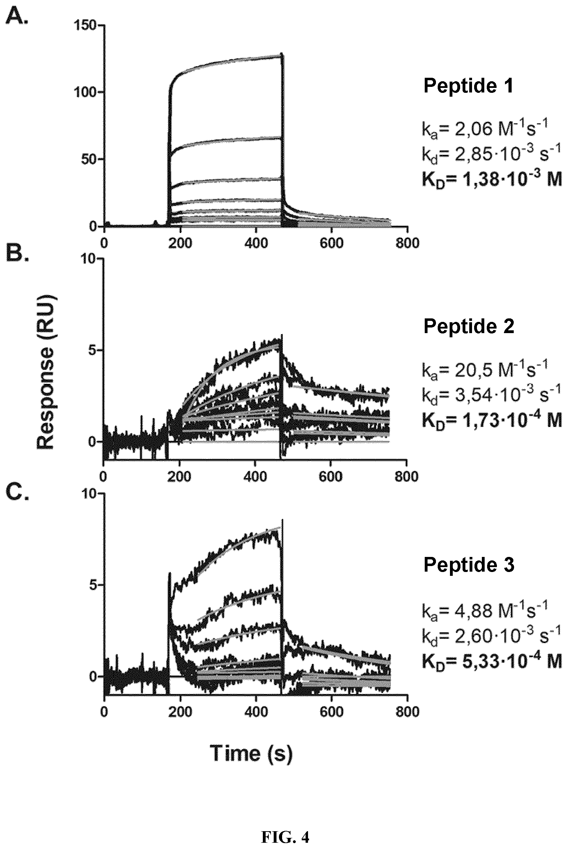

Figure 4 shows the SPR sensograms of peptides of the invention binding to immobilized human His6-TWEAK protein: A) peptide 1, B) peptide 2, and C) peptide 3. The experimental data (black lines) were globally fit to a 1:1 binding model (grey lines) using BIAevaluation, as described in the examples, to determine the indicated kinetic rate and affinity constants. Data are representative of at least three independent experiments.

### DETAILED DESCRIPTION OF THE INVENTION

### Peptides of the invention

In the first aspect, the present invention relates to a peptide selected from the group consisting of TTDGSTGDDLQA (SEQ ID NO: 1), SWYDHMAGDSSY (SEQ ID NO: 2), SYTDVQDS (SEQ ID NO: 3) and DNGGGNTDIYSE (SEQ ID NO: 4) or a pharmaceutically acceptable salt thereof.

In this description the abbreviations used for amino acids follow the recommendations of the 1983 IUPAC-IUB Commission of Biochemical Nomenclature specified in Eur. J. Biochem., (1984), 138, 937.

Thus, for example, S or Ser represents NH₂-CH(CH₂-OH)-COOH, S- or Ser- represents NH₂-CH(CH₂-OH)-CO-, -S or -Ser represents -NH-CH(CH₂-OH)-COOH, and -S- or - Ser- represents -NH-CH(CH₂-OH)-CO-. Therefore, the hyphen, which represents the peptide bond, eliminates the OH in the terminal carboxyl group of the amino acid (represented here in the conventional non-ionized form) when situated to the right of the symbol, and eliminates the H of the terminal amino group of the amino acid when situated to the left of the symbol; both modifications can be applied to the same symbol.

The aminoacids are named using the conventional nomenclature in one and three letter codes, as follows:
- S, Ser or serine,
- W, Trp or tryptophan,
- Y, Tyr or tyrosine,
- D, Asp or aspartic acid,
- H, His or histidine,
- M, Met or methionine,
- A, Ala or alanine,
- G, Gly or glycine,
- T, Thr or threonine,
- L, Leu or leucine,
- Q, Gln or glutamine,
- V, Val or valine,
- N, Asn, or asparagine,
- I, Ile or isoleucine, and
- E, Glu or glutamic acid.

The term "pharmaceutically acceptable salts" means a salt recognized for its use in human beings, and includes salts used to form base addition salts, either inorganic or organic, or acid addition salts, either inorganic or organic. The nature of the salt is not critical, provided that it is cosmetically or pharmaceutically acceptable. The pharmaceutically acceptable salts of the peptides of the invention can be obtained by the conventional methods, well known in the prior art [Berge S.M. et al., "Pharmaceutical Salts", (1977), J. Pharm. Sci., 66, 119].

The peptides may be prepared by conventional peptide synthesis using the procedures described in the examples.

The preparation of salts can be carried out by methods known in the art. For instance, cosmetically acceptable salts of compounds provided herein may be acid addition salts, base addition salts or metallic salts, and they can be synthesized from the parent compound which contains a basic or acidic moiety by conventional chemical methods. Generally, such salts are, for example, prepared by reacting the free acid or base forms of these compounds with a stoichiometric amount of the appropriate base or acid in water or in an organic solvent or in a mixture of the two. Generally, non-aqueous media like ether, ethyl acetate, ethanol, isopropanol or acetonitrile are preferred. Examples of the acid addition salts include mineral acid addition salts such as, for example, hydrochloride, hydrobromide, hydroiodide, sulphate, nitrate, phosphate, and organic acid addition salts such as, for example, acetate, maleate, fumarate, citrate, oxalate, succinate, tartrate, malate, mandelate, methanesulphonate and p-toluenesulphonate. Examples of the alkali addition salts include inorganic salts such as, for example, ammonium, and organic alkali salts such as, for example, ethylenediamine, ethanolamine, N,N-dialkylenethanolamine, triethanolamine, glucamine and basic aminoacids salts. Examples of the metallic salts include, for example, sodium, potassium, calcium, magnesium, aluminium and lithium salts.

In a preferred embodiment, the peptide of the invention is TTDGSTGDDLQA (SEQ ID NO: 1).

In another preferred embodiment, the peptide of the invention is SWYDHMAGDSSY (SEQ ID NO: 2).
In another preferred embodiment, the peptide of the invention is SYTDVQDS (SEQ ID NO: 3).

In another preferred embodiment, the peptide of the invention is DNGGGNTDIYSE (SEQ ID NO: 4).

### Compositions of the invention

The peptides of the invention may be administered in a composition comprising said peptides and a pharmaceutically acceptable excipient.

Thus in a second aspect, the present invention relates to a composition comprising a peptide as defined in the first aspect and a pharmaceutically acceptable excipient.

By "pharmaceutically acceptable excipient" is meant herein a material that is not biologically or otherwise undesirable, i.e. the material may be incorporated into a pharmaceutical composition administered to a patient without causing any undesirable biological effects or interacting in a deleterious manner with any of the other components of the composition in which it is contained.

The term "excipient" refers to a diluent, adjuvant, carrier, or vehicle with which the active ingredient is administered and includes any such materials known in the art that are nontoxic and do not interact with other components of a pharmaceutical composition or drug delivery system in a deleterious manner. Such pharmaceutical excipients can be sterile liquids, such as water and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as carriers, particularly for injectable solutions. Suitable pharmaceutical carriers are described in "Remington's Pharmaceutical Sciences" by E.W. Martin.

For its administration to a subject, such as a mammal, e.g., a human, in need of treatment, the pharmaceutical composition of the invention may be administered by any appropriate route, such as, oral (e.g., oral, sublingual, etc.), parenteral (e.g., subcutaneous, intramuscular, intravenous, etc.), respiratory (e.g., inhalation or insufflation), rectal, or local administration (e.g. implants, transdermal, etc.).

Examples of pharmaceutical compositions include any solid (tablets, pills, capsules, granules etc.) or liquid (solutions, suspensions or emulsions) composition for oral or parenteral administration. The mentioned formulations will be prepared using standard methods such as those described or referred to in the European and US Pharmacopoeias and similar reference texts. The pharmaceutical compositions may also comprise the peptides of the invention encapsulated in nanoparticles or liposomes.

The pharmaceutical compositions may be in oral form, either solid or liquid. Suitable dose forms for oral administration may be tablets, capsules, syrups or solutions and may contain conventional excipients known in the art such as binding agents, for example syrup, acacia, gelatine, sorbitol, tragacanth, or polyvinylpyrrolidone; fillers, for example lactose, sugar, maize starch, calcium phosphate, sorbitol or glycine; tableting lubricants, for example magnesium stearate; disintegrants, for example starch, polyvinylpyrrolidone, sodium starch glycolate or microcrystalline cellulose; or pharmaceutically acceptable wetting agents such as sodium lauryl sulfate. The solid oral compositions may be prepared by conventional methods of blending, filling or tablet pharmaceutical production. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating.

The pharmaceutical compositions may also be adapted for parenteral administration and local administration, such as sterile solutions, suspensions or lyophilized products in the appropriate unit dosage form. Adequate excipients can be used, such as bulking agents, buffering agents or surfactants.

In particular, local administration refers to the administration of the disclosed peptides or compositions to the area in need of treatment. Examples include local infusion during surgery; topical application, by local injection; by a catheter; by a suppository; or by an implant.

The peptides and compositions of this invention may be used with other drugs to provide a combination therapy, in particular in combination with another chemotherapeutic agent. The other drugs may form part of the same composition, or be provided as a separate composition for administration at the same time or at different time. In this sense, whenever it is said that a composition comprises a certain drug combination, it is meant that the composition comprises the drugs contained in the drug combination, i.e. the drugs of the drug combination form part of the composition.

### Uses

The peptides of the invention are able to inhibit the TWEAK-Fn14 signaling pathway, which is involved in the pathways responsible for tumor cell invasion and migration, as well as proliferation and angiogenesis.

Thus, in a third aspect the present invention relates to a peptide as defined in the first aspect or a composition as defined in the second aspect for use in medicine.

In a fourth aspect the present invention relates to a peptide as defined in the first aspect or a composition as defined in the second aspect for use in the treatment and/or prevention of a Fn-14 positive solid cancer.

This aspect may also be formulated as the use of a peptide as defined in the first aspect or a composition as defined in the second aspect in the manufacture of a medicament for the treatment and/or prevention of an Fn-14 positive solid cancer.

This aspect may also be formulated as a method of treatment and/or prevention of a Fn-14 positive solid cancer comprising administering to a subject in need thereof a peptide as defined in the first aspect or a composition as defined in the second aspect.

As used herein, the terms "treat", "treatment" and "treating" refer to the reduction or amelioration of the progression, severity and/or duration of a disease or disorder, such as a solid tumor, or the amelioration of one or more symptoms (preferably, one or more discernible symptoms) related a disease or disorder, such as a solid tumor, e.g. reduction or stabilization of tumor size or cancerous cell count.

As used herein, the terms "prevent", "prevention" and "preventing" refer to the reduction in the risk of acquiring or developing a given disease or disorder, e.g., a solid tumor, or the reduction or inhibition of the recurrence or a disease or disorder, e.g., a solid tumor.

As used herein, the terms "subject", "patient" are used interchangeably. The terms "subject" and "patient" refer to an animal (e.g., a bird such as a chicken, quail or turkey, or a mammal), preferably a mammal including a non-primate (e.g., a cow, pig, horse, sheep, rabbit, guinea pig, rat, cat, dog, and mouse) and a primate (e.g., a monkey, chimpanzee and a human), and more preferably a human. In one embodiment, the subject is a non-human animal such as a farm animal (e.g., a horse, cow, pig or sheep), or a pet (e.g., a dog, cat, guinea pig or rabbit). In another embodiment, the subject is a human.

The term "cancer" or "tumor", as used herein, refers to a broad group of diseases involving unregulated cell growth and which are also referred to as malignant neoplasms. The term is usually applied to a disease characterized by uncontrolled cell division (or by an increase of survival or apoptosis resistance) and by the ability of said cells to invade other neighboring tissues (invasion) and spread to other areas of the body where the cells are not normally located (metastasis) through the lymphatic and blood vessels, circulate through the bloodstream, and then invade normal tissues elsewhere in the body. Depending on whether or not they can spread by invasion and metastasis, tumors are classified as being either benign or malignant: benign tumors are tumors that cannot spread by invasion or metastasis, i.e., they only grow locally; whereas malignant tumors are tumors that are capable of spreading by invasion and metastasis. Biological processes known to be related to cancer include angiogenesis, immune cell infiltration, cell migration and metastasis. Cancers usually share some of the following characteristics: sustaining proliferative signaling, evading growth suppressors, resisting cell death, enabling replicative immortality, inducing angiogenesis, and activating invasion and eventually metastasis. Cancers invade nearby parts of the body and may also spread to more distant parts of the body through the lymphatic system or bloodstream. Cancers are classified by the type of cell that the tumor cells resemble, which is therefore presumed to be the origin of the tumor.

As used herein, the term "solid cancer" or "solid tumor" refers to an abnormal growth of body tissue cells other than blood, bone marrow or lymphatic cells.

Preferred solid cancers are carcinomas and glioblastoma.

"Carcinomas" are cancers derived from epithelial cells. This group includes many of the most common cancers, particularly in the aged, and include lung cancer, colorectal cancer, pancreatic cancer, larynx cancer, tongue cancer, prostate cancer, breast cancer, ovarian cancer, liver cancer, head and neck cancer, esophageal cancer, renal cancer, endometrial cancer, gall bladder cancer, bladder cancer and gastric cancer. Carcinomas are of two types: adenocarcinoma and squamous cell carcinoma. Adenocarcinoma develops in an organ or gland and squamous cell carcinoma originates in squamous epithelium. Adenocarcinomas may affect mucus membranes and are first seen as a thickened plaque-like white mucosa. These are rapidly spreading cancers.

"Glioblastoma" is a fast-growing type of central nervous system tumor that forms from glial (supportive) tissue of the brain and spinal cord and has cells that look very different from normal cells. Glioblastoma usually occurs in adults and affects the brain more often than the spinal cord. It is also called GBM, glioblastoma multiforme, and grade IV astrocytoma.

Examples of solid tumors are brain cancer, breast cancer, lung cancer, melanoma, liver cancer, colorectal cancer, esophageal cancer, bladder cancer pancreatic cancer, ovarian cancer, prostate cancer, neuroblastoma, larynx cancer, tongue cancer, renal cancer, endometrial cancer, gall bladder cancer, and gastric cancer.

The term "Fn-14 positive cancer" refers to a cancer, which is characterized by an overexpression of fn-14 protein or FN-14 gene.

As previously explained, "Fn-14" is a type I transmembrane protein [Chicheportiche et al., J Biol Chem., 1997, 272, 32401-32410; Wiley et al., Immunity, 2001, 15, 837-846; Swiss-Prot Q9NP84]. Fn14 contains the extracellular domain that binds to TWEAK as well as the cytoplasmic tail region essential for signal transduction [Winkles, Nat Rev Drug Discov., 2008, 7, 411-425].

Fn-14 expression levels may be determined by standard assays known in the art.

By way of example, Fn-14 levels can be quantified, for example, by using antibodies capable of binding to fn14 (or fragments thereof that contain an antigenic determinant), and the subsequent quantification of the complexes formed. The antibodies used in these assays may or may not be labeled. Illustrative examples of markers that can be used include radioactive isotopes, enzymes, fluorophores, chemiluminescent reagents, enzyme substrates or cofactors, enzyme inhibitors, particles, dyes, etc. There is a wide variety of known assays that can be used that use unlabeled antibodies (primary antibody) and labeled antibodies (secondary antibody). These techniques include Western blot or Western blot, ELISA (enzyme-linked immunosorbent assay), RIA (radioimmunoassay), competitive EIA (competitive enzyme immunoassay), DAS-ELISA (double antibody sandwich ELISA), immunocytochemical and immunohistochemical techniques, techniques based on the use of biochips or protein microarrays that include specific antibodies or tests based on colloidal precipitation in formats such as dipsticks. Other ways to detect and quantify proteins include affinity chromatography techniques, ligand binding assays, etc. When using an immunological method, any antibody or reagent known to bind to the protein with high affinity can be used to detect the amount of it.

In a particular embodiment, the Fn-14 positive cancer refers to a cancer having an overexpression of Fn-14 protein.

On the other hand, the quantification of the expression level of the FN14 gene can also be performed by quantifying the expression levels by measuring the levels of the FN14 messenger RNA. To this end, the biological sample can be treated to physically or mechanically disintegrate the tissue or cell structure, releasing the intracellular components into an aqueous or organic solution to prepare the nucleic acids.

Any conventional method can be used to detect and quantify the levels of mRNA encoded by the FN14 gene or its corresponding cDNA. By way of example, the levels of mRNA encoded by said gene can be quantified using conventional methods, for example, methods that comprise the amplification of mRNA and the quantification of the product of the amplification of said mRNA, and use of specific probes for the mRNA of the FN14 gene or its corresponding cDNA, preferably by quantitative real-time PCR using an appropriate marker. In a particular embodiment, the quantification of the expression levels of the FN14 gene is performed by means of a quantitative polymerase chain reaction (PCR) or an array of DNA or RNA or by nucleotide hybridization techniques.

In another particular embodiment, the Fn-14 positive cancer refers to a cancer having an overexpression of FN-14 gene.

In a particular embodiment, FN-14 expression is determined by immunohistochemical techniques following the procedure described in Martinez-Aranda et al. [Oncotarget. 2015, 6(42), 44254-44273; Frontiers in Oncology, 2017, 7, 283]. Immunoreactivities were classified by estimating the percentage of tumor cells showing characteristic staining (from "undetectable" or 0%, to homogeneous staining or 100%). In a particular embodiment, cells showing more than 25% of expressing cells was considered as positive. [Sanz-Pamplona et al., Am J Pathol, 2011, 179(2), 564-579].

In another particular embodiment, FN-14 expression is determined by performing quantitative PCR (qPCR) and analysis of expression results after initiating analyzes with standard qPCR curves using the ACTB assay (gene encoding beta-actin, cytoskeleton component awaiting positive amplification and elevated in all cases) with a subgroup of all types of samples (cell lines, mouse xenografts of tumors and clinical samples) to determine the efficiency of qPCR. The predesigned TaqMan assays had an efficiency of 100% ± 10% and through the use of specific software the cut-off points were established for the classification of patients with overexpressed FN14 at 1.035 (Sensitivity: 66.7%, Specificity: 86.4 %).

The second stage of quantification involves comparing the level of expression obtained with respect to a reference sample. In general, typical reference samples will be obtained from subjects who are clinically well documented.

The term "reference sample" or "control sample" is selected depending on the technique, tools and manufacturer's instructions used in order to determine the level of expression FN14. In a particular embodiment, the reference sample is taken from a healthy subject, although it may also be taken from other subjects, including subjects who do not have a clinical history of a disease or condition wherein cells expressing FN-14 participate, the same subject but from a tissue or part thereof which is under a normal physiological condition or healthy. In a particular embodiment, the reference sample is a sample from a healthy subject. In another particular embodiment, the reference sample is a sample from an individual who does not have a clinical history of a disease or condition wherein cells expressing FN-14 participate. In another particular embodiment, the reference sample is a sample from the same subject but from a tissue or part thereof which is healthy. In another particular embodiment, the reference sample are cells from the triple negative MDA-MB 435 (435-P) cell line [Schackert et al., Int J Cancer, 1989, 44, 892-897]. A positive control, for example a cell line that expresses FN-14 can additionally be used if desired.

"Increased expression levels", "high expression levels" or "overexpression" refers to an expression level of Fn-14 higher than that of the reference sample. Preferably when the sample under study has at least 1.1 times, 1.5 times, 5 times, 10 times, 20 times, 30 times, 40 times, 50 times, 60 times, 70 times, 80 times, 90 times, 100 times or even more with respect to the reference value FN14. While "decreased expression levels" or "low expression levels" refer to the expression level of Fn-14 being lower than that of the reference sample. In particular, a sample can be considered to have low levels of Fn-14 expression when the expression levels in the subject sample have been reduced at least 1.1 times, 1.5 times, 5 times, 10 times, 20 times, 30 times, 40 times, 50 times, 60 times, 70 times, 80 times, 90 times or 100 times with respect to the Fn-14 reference value.

Cancers that can be treated or prevented by the peptides or compositions of the present invention are Fn-14 positive solid cancers selected from the group consisting of brain cancer, breast cancer, lung cancer (in particular non-small cell lung cancer (NSCLC), more particularly squamous cell carcinoma), melanoma, liver cancer, colorectal cancer, esophageal cancer, bladder cancer, pancreatic cancer, ovarian cancer, prostate cancer and neuroblastoma. Preferably, the solid cancer is brain cancer. Even more preferably, the brain cancer is metastatic brain cancer.

In particular, the Fn-14 positive solid cancers referred to above are TWEAK-dependent.

The term "metastasis" or "metastatic", as used herein, refers to the spread of a cancer from one organ or tissue to another non-adjacent organ or tissue. Cancer cells can break away, leak, or spill from a primary tumor and enter lymphatic and blood vessels, circulate throughout the bloodstream, and be deposited elsewhere in the body. This occurrence is referred to as "metastasis". Metastasis is one of the hallmarks of malignancy of cancers.

Furthermore, the peptides for use according to the present invention are applicable to a subject who suffers from any of the above mentioned solid cancers at stages 0, I, II, III or IV. Stage 0 is used to describe cancer in situ; these cancers are still located in the place they started and have not invaded nearby tissues. Stage I is a small cancer or tumor that has not grown deeply into nearby tissues and has not spread to the lymph nodes or other parts of the body. Stage II and III indicate cancers or tumors that are larger in size, have grown more deeply into nearby tissue, and have spread to lymph nodes, but not to other parts of the body. Stage IV means that the cancer has spread to other organs or parts of the body. It may also be called advanced or metastatic cancer.

A metastatic brain cancer refers to metastasis in the brain of a cancer (primary tumor) from an organ or tissue other than brain.

In a preferred embodiment, the brain metastasis is of a Fn-14 positive cancer (primary tumor) selected from the group consisting of breast cancer, lung cancer, melanoma, colon cancer, kidney cancer and melanoma; more preferably selected from the group consisting of breast cancer, lung cancer, colon cancer and melanoma; even more preferably brain metastasis of breast cancer, lung cancer and colon cancer; the most preferred is brain metastasis of breast cancer.

In another preferred embodiment, the Fn-14 positive cancer to be treated or prevented by the peptides or compositions of the present invention is a glioblastoma. The peptides of the present invention are particularly useful for the prevention.

Cancer-induced cachexia refers to a complex metabolic syndrome associated with underlying illness, in the present case, with cancer, and is characterized extreme weight loss and muscle wasting. Cancer-induced cachexia is characterized by systemic inflammation, negative protein and energy balance, and an involuntary loss of lean body mass, with or without wasting of adipose tissue.

Thus, in a further aspect the present invention relates to a peptide as defined in the first aspect or a composition as defined in the second aspect for use in the treatment and/or prevention of cancer-induced cachexia.

This aspect may also be formulated as the use of a peptide as defined in the first aspect or a composition as defined in the second aspect in the manufacture of a medicament for the treatment and/or prevention of cancer-induced cachexia.

This aspect may also be formulated as a method of treatment and/or prevention of cancer-induced cachexia comprising administering to a subject in need thereof a peptide as defined in the first aspect or a composition as defined in the second aspect.

As used herein, the term "effective amount" refers to an amount of a peptide of this invention which is sufficient to reduce or ameliorate the severity, duration, progression, or onset of a the solid cancers or cancer-induced cachexia described above, prevent the advancement of a the solid cancers or cancer-induced cachexia described above, cause the regression of the solid cancers or cancer-induced cachexia described above, prevent the recurrence, development, onset or progression of a symptom associated with the solid cancers or cancer-induced cachexia described above, or enhance or improve the prophylactic or therapeutic effect(s) of another therapy. The precise amount of peptide administered to a subject will depend on the mode of administration, the type and severity of the solid cancer and on the characteristics of the subject, such as general health, age, sex, body weight and tolerance to drugs. It will also depend on the degree, severity and type of cell proliferation, and the mode of administration. The skilled artisan will be able to determine appropriate dosages depending on these and other factors. When co-administered with other agents, e.g., when co-administered with another chemotherapeutic agent, an "effective amount" of the second agent (chemotherapeutic agent) will depend on the type of drug used. Suitable dosages are known for approved agents and can be adjusted by the skilled artisan according to the condition of the subject, the type of condition(s) being treated and the amount of a peptide of the invention being used. In cases where no amount is expressly noted, an effective amount should be assumed.

Non-limiting examples of an effective amount of a peptide of the invention is provided herein below: a dose (expressed as amount of compound per kg of body weight) of at least about 150 µg/kg, preferably at least about 250 µg/kg, at least about 500 µg/kg, at least about 1 mg/kg, at least about 5 mg/kg, at least about10 mg/kg, at least about 25 mg/kg, at least about 50 mg/kg, at least about 75 mg/kg, at least about 100 mg/kg, at least about 125 mg/kg, at least about 150 mg/kg, or at least about 200 mg/kg or more of a peptide of the invention. The peptides of the invention may be administered once every day, preferably, once every 2 days, once every 3 days, once every 4 days, once every 5 days, once every 6 days, once every 7 days, once every 8 days, once every 10 days, once every two weeks, once every three weeks, or once a month.

The following examples represent specific embodiments of the present invention. They do not intend to limit in any way the scope of the invention defined in the present description.

### EXAMPLES

### Example 1. Peptide synthesis and characterization

Peptides were synthesized in C-terminal carboxamide form by Fmoc solid-phase peptide synthesis on a H-Rink Amide-ChemMatrix resin of 0.50 mmol/g substitution (PCAS BioMatrix, Quebec, Canada) at a 0.05 mmol scale in a Prelude automated synthesizer (Protein Technologies, Tucson, AZ). Side chains of trifunctional residues were protected with TFA-labile t-butyl (Glu, Ser, Thr) and trityl (Asn, Gln, His) groups. Couplings were systematically performed with a 5-fold excess of Fmoc-amino acid in the presence of N,N,N',N'- tetramethyluronium hexafluorophosphate (5 eq) and N,N-diisopropylethylamine (10 eq) with DMF as solvent. Fmoc removal was done with piperidine/DMF (20:80 v/v) followed by DMF washes. Cleavage and deprotection of the peptide resins was done with TFA-water-triisopropylsilane (95:2.5:2.5, v/v/v, 90 min, r.t.). Peptides were isolated by precipitation with cold diethyl ether and centrifugation, then solubilized in water and lyophilized.

The synthetic crude products were analyzed by analytical RP-HPLC and LC-MS and purified by preparative RP-HPLC. Analytical RP-HPLC was performed on a LC-20AD instrument (Shimadzu, Kyoto, Japan) fitted with a Luna C18 column (4.6 mm × 50 mm, 3 µm; Phenomenex, Torrance, CA, USA) using linear gradients of solvent B (0.036% TFA in ACN) into A (0.045% TFA in H₂O) over 15 min, at 1 mL/min flow rate and with UV detection at 220 nm. Preparative RPHPLCwas performed on a LC-8 instrument (Shimadzu) fitted with a Luna C18 column (21.2 mm × 250 mm, 10 µm; Phenomenex), using linear gradients of solvent D (0.1% TFA in ACN) into C (0.1% TFA in H₂O) over 30 min, with a flow rate of 25 mL/min. MS analysis was performed on a LC-MS 2010EV instrument (Shimadzu) fitted with an XBridge C18 column (4.6 mm × 150 mm, 3.5 µm, Waters, Cerdanyola del Vallès, Spain), eluting with linear gradients of F (0.08% formic acid (FA) in ACN) into E (0.1% FA in H₂O) over 15 min at 1 mL/min flow rate. Fractions of >95% HPLC purity and with the expected mass by LC-MS were pooled and lyophilized.

The analytical characterization of the peptides is provided in the table below:

| Peptide Sequence | MW (Da)^{a} | RP-HPLC method^{b} | tR (min) | Purity (%)^{c} |
|---|---|---|---|---|
| Peptide 1: SWYDHMAGDSSY (SEQ ID NO: 2) | 1417.48 | 5-60% B | 5.93 | 95 |
| Peptide 2: TTDGSTGDDLQA (SEQ ID NO: 1) | 1179.16 | 5-60% B | 3.43 | 96 |
| Peptide 3: SYTDVQDS (SEQ ID NO: 3) | 912.91 | 5-60% B | 3.47 | 93 |
| Peptide 4: DNGGGNTDIYSE (SEQ ID NO: 4) | 1240.21 | 5-60% B | 6.50 | 88 |

| | | | | |
|---|---|---|---|---|
| ^{a} Identity was established by HPLC-MS. ^{b} Analytical RP-HPLC was performed using linear gradients of solvent B (0.036% TFA in ACN) into A (0.045% TFA in H₂O) over 15 min at 1 mL/min flow rate and with UV detection at 220 nm. ^{c} Peptide purity was calculated using the percentage of peak area at the chromatographic profile. | | | | |

### Example 2. Biological activity

### Cell lines

BRV5 is a highly metastatic brain cell line, obtained in one of our laboratories by primary culture from a brain of a nude mouse inoculated with human 435-Br1 brain metastatic cells, as previously described [Martinez-Aranda et al., Int J Mol Sci., 2013, 14, 8306-8327]. The triple negative MDA-MB 435 (435-P) cell line [Schackert et al., Int J Cancer, 1989, 44, 892-897] is a useful human breast cancer model that expresses both epithelial and melanocytic markers [Chambers, Cancer Res., 2009, 69, 5292-5293], and was included as the parental cell from which 435-Br1 metastatic variant was initially originated (both cell lines kindly provided by Dr Fabra at IDIBELL Institute, Hospitalet de Llobregat, Spain). Finally, HT29 human colon carcinoma cells obtained from the European Type Culture Collection (ECACC 91072201) were used as a positive control of TWEAK dependent Fn14 over-expressing cells to evaluate TWEAK-Fn14 axis of brain metastatic variants.

### Cell cultures conditions and treatments

Appropriate cell culture conditions were established as the controls needed in the expression of genes with and without peptides and with and without TWEAK activation. The protocol started with the seeding of 125,000 cells/well in 500 µl of culture media supplemented with 10% fetal bovine serum (CM+FBS10%) in 24-well dishes for 24 h before removing FBS (CM-FBS). After overnight starvation in CM-FBS culture, different peptide treatment conditions (at final concentration of 450 µM) were established in the presence or absence of TWEAK (25 ng/mL final concentration) (Peprotech EC, Ltd, U.K.). Some cells were treated with peptides at a single, unique, initial dose (time 0) while other were treated with repeated doses to account for the effect cell-culture degradation. These include a repeated treatment every hour (i.e. time 0,1,3,4,5), every two hours (time 0, 2 and 4) and every three hours (time 0 and 3). At time 6 (after six hours) cells were lisated and RNA extracted for transcriptomic analyses (see next). Cell were cultured for six hours as this was the time needed to achieve the highest upregulation of the TWEAK-Fn14 axis upon TWEAK treatment.

### Transcriptomic analysis

RNA from cells was extracted 6 h after treatments by direct lysis on the well. The RNeasy Micro kit (Qiagen, Germany) was used to isolate total RNA from cell lysate following manufacturer's protocols including an additional step of digestion with DNase I (Qiagen, Germany). Total RNA concentration was measured in a NanoDrop 1000 spectrophotometer (Thermo Scientific, Waltham, MA). The RNA extracted from treated and untreated cells (see above) was used as a proxy for gene expression.

The quantitative RT-PCR was performed using Fluidigm Dynamic Array FlexSix array with the specific primers for gene expression (Delta Genes Assays, Wet lab tested on Dynamic Array IFC) designed to RefSeq for detection by pPCR EvaGreen assay (Dynamic Array FlexSix, Fluidigm Corporation). Each Flex Six IFC has a total of six independent partitions. Each partition has a 12 × 12 format (12 assay inlets and 12 sample inlets) and will be run independently as a separate experimental run. The dynamic array was loaded with the sample and assay mixtures via the appropriate inlets using an IFC controller and the chip placed in the BioMark Instrument according to the protocol GE Flex Six PCR+Melt v1. Data was analyzed with Real-Time PCR Analysis Software in the BIOMARK instrument (Fluidigm Corporation) to obtain Ct and delta Ct values.

The level of expression of 11 genes were investigated; 9 on the signaling pathways downstream from the TWEAK-Fn14 axis, and two housekeeping genes. The downstream genes were: TNF receptor-associated factor 2 (TRAF2), IAPS (BIRC2), NIK (MAP4K4), kappa light polypeptide gene enhancer in B-cells 2 (NFkB2), P38 (MAPK14), interleukin 6 (IL-6), GRP94 (HSP90B1), Fn14 (TNFR2F12A) and JUN proto-oncogene (JNK). The two housekeeping genes were the asparaginyl-tRNA synthetase 2 (NARS2) and mixed-lineage leukemia AF10 (MLLT10) gene.

The genes were selected due to their properties as described below:
*TRAF2: TNF receptor-associated factor* 2. Regulates activation of NF-kappa-B and JNK and plays a central role in the regulation of cell survival and apoptosis. Required for normal antibody isotype switching from IgM to IgG. Has E3 ubiquitin-protein ligase activity and promotes 'Lys-63'-linked ubiquitination of target proteins, such as BIRC3, RIPK1 and TICAM1. Is an essential constituent of several E3 ubiquitin-protein ligase complexes, where it promotes the ubiquitination of target proteins by bringing them into contact with other E3 ubiquitin ligases. Regulates BIRC2 and BIRC3 protein levels by inhibiting their autoubiquitination and subsequent degradation; this does not depend on the TRAF2 RING-type zinc finger domain. Plays a role in mediating activation of NF-kappa-B by EIF2AK2/PKR. In complex with BIRC2 or BIRC3, promotes ubiquitination of IKBKE.
*TNFRSF12A (Fn14): Tumor necrosis factor receptor superfamily member 12A.* Receptor for TNFSF12/TWEAK. Weak inducer of apoptosis in some cell types. Promotes angiogenesis and the proliferation of endothelial cells. May modulate cellular adhesion to matrix proteins.
*MAP4K4: Mitogen-activated protein kinase kinase kinase kinase 4.* Serine/threonine kinase that may play a role in the response to environmental stress and cytokines such as TNF-alpha. Appears to act upstream of the JUN N-terminal pathway. Phosphorylates SMAD1 on Thr-322.
*NFkB2: Nuclear factor NF-kappa-B p100 subunit.* NF-kappa-B is a pleiotropic transcription factor present in almost all cell types and is the endpoint of a series of signal transduction events that are initiated by a vast array of stimuli related to many biological processes such as inflammation, immunity, differentiation, cell growth, tumorigenesis and apoptosis. F-kappa-B is controlled by various mechanisms of post-translational modification and subcellular compartmentalization as well as by interactions with other cofactors or corepressors.
*MAPK14: Mitogen-activated protein kinase 14.* Serine/threonine kinase which acts as an essential component of the MAP kinase signal transduction pathway. MAPK14 is one of the four p38 MAPKs which play an important role in the cascades of cellular responses evoked by extracellular stimuli such as proinflammatory cytokines or physical stress leading to direct activation of transcription factors.
*JUN: Transcription factor AP-1.* Transcription factor that recognizes and binds to the enhancer heptamer motif 5'-TGA[CG]TCA-3'. Promotes activity of NR5A1 when phosphorylated by HIPK3 leading to increased steroidogenic gene expression upon cAMP signaling pathway stimulation
*IL6: Interleukin-6.* Cytokine with a wide variety of biological functions. It is a potent inducer of the acute phase response. Plays an essential role in the final differentiation of B-cells into Ig-secreting cells Involved in lymphocyte and monocyte differentiation. Acts on B-cells, T-cells, hepatocytes, hematopoietic progenitor cells and cells of the CNS.
*HSP90B1: Endoplasmin.* Molecular chaperone that functions in the processing and transport of secreted proteins. When associated with CNPY3, required for proper folding of Toll-like receptors. Functions in endoplasmic reticulum associated degradation (ERAD)
*BIRC2: Baculoviral IAP repeat-containing protein 2.* Multi-functional protein which regulates not only caspases and apoptosis, but also modulates inflammatory signaling and immunity, mitogenic kinase signaling, and cell proliferation, as well as cell invasion and metastasis. Acts as an E3 ubiquitin-protein ligase regulating NF-kappa-B signaling and regulates both canonical and non-canonical NF-kappa-B signaling by acting in opposite directions: acts as a positive regulator of the canonical pathway and suppresses constitutive activation of non-canonical NF-kappa-B signaling.

The normalized level of expression of genes were obtained as follows. RNA from cells was extracted by direct lysis. The RNeasy Micro kit (Qiagen, Germany) was used to isolate total RNA from cell lysate following manufacturer's protocols including an additional step of digestion with DNase I (Qiagen, Germany). Total RNA concentration was measured in a NanoDrop 1000 spectrophotometer (Thermo Scientific, Waltham, MA).The quantitative RT-PCR was performed using Fluidigm Dynamic Array FlexSix array with the specific primers for gene expression (Delta Genes Assays, Wet lab tested on Dynamic Array IFC) designed to RefSeq for detection by pPCR EvaGreen assay (Dynamic Array FlexSix, Fluidigm Corporation). Data was analyzed with Real-Time PCR Analysis Software in the BIOMARK instrument (Fluidigm Corporation) to obtain Ct and delta Ct values and the expression of housekeeping gene NARS2 was used to normalize (Zscores) all values and quantify gene expression.

### Surface plasmon resonance (SPR) analysis

Binding assays were performed at 25°C in a Biacore 3000 instrument (GE Healthcare, Uppsala, Sweden) using nitrilotriacetic (NTA) sensor chips (GE Healthcare) and HBS-EP (0.01 M HEPES pH 7.4, 0.15 M NaCl, 3 mM EDTA, 0.005 % v/v Surfactant P20) as running buffer. Histidine-tagged human TWEAK (His6-TWEAK) protein (R&D Systems, Minneapolis, MN, USA) was covalently immobilized on the sensor surface using a capture coupling method 28. Briefly, the NTA surface was first stabilized by injection of regeneration buffer (20 µL EDTA-containing running buffer at 20 µL/min) followed by Ni²⁺ binding to the surface (40 µL of 500 µM NiCl₂ in running buffer at 20 µL/min). The carboxymethyl dextran surface was then activated for primary amine coupling by injecting coupling solution (30 µL of a 1:1 N-hydroxysuccinimide (NHS)/1-ethyl-3-[3-(dimethylamino)-propyl]carbodiimide hydrochloride (EDC) mixture at 5 µL/min). His6-TWEAK protein in pH 7.4 running buffer was then injected over the surface (60 µL of 10 µg/mL His6-TWEAK at 5 µL/min). To block uncoupled primary amines on the sensor surface, ethanolamine was passed over the surface (35 µL 1M ethanolamine at 5 µL/min). Ligand surfaces were finally conditioned with a 20 µL injection of regeneration solution containing 350 mM EDTA at 20 µL/min. Immobilization levels ranged from 1500 to 7000 resonance units (RUimmob) to test different ligand densities. A blank flow cell on the chip was created by simple injection of 20 µL of regeneration buffer injected over the sensor surface.

Binding experiments using a monoclonal anti-TWEAK antibody (Abcam ref. 199405, Cambridge, UK) were run to ascertain adequate functionalization of the immobilized surface. Peptides at two-fold serial dilutions from 800 µM to 0 µM in running buffer were flown over the chip for 5 min at 35 µL/min. The dissociation phase was monitored for 10 min at the same flow rate and followed by a regeneration step with 10 µL of 18 mM NaOH. Specific binding responses for each channel were obtained by double referencing; subtracting non-specific binding to the blank flow cell plus an internal reference standard, namely buffer injection response. Each sample was run in triplicate. The experimental data were fitted to a 1:1 Langmuir binding model by the BIAevaluation software version 4.1 (GE Healthcare, Uppsala, Sweden).

### Results

Fn14 expression is strongly enhanced in human colon carcinoma cells HT29 compared to normal cells and other different transformed tissues. We used these cells as a prototype to evaluate the relative expression of Fn14 in brain metastatic cells. Immunofluorescent analyses of cell cultures showed Fn14 protein distributed in cellular membrane and cytoplasm (Figure 1) with similar intensity and localization in HT29 and BRV5 cells. In contrast, breast cancer 435-P parental cells, from which BRV5 was derived, had lower staining intensity, either membrane or cytoplasm, indicating less Fn14 protein expression.

Since transcriptional activation of Fn14 expression can be induced in cancer cells by the TWEAK-Fn14 interaction, either in a paracrine or autocrine manner [Winkles, Nat Rev DrugDiscov., 2008, 7, 411-425], we investigated whether TWEAK transactivates Fn14 (TNFR2F12A) in brain metastatic cells. We primed cells with TWEAK and analyzed TNFR2F12A gene expression including in the analysis 435-Br1 cells, the first generation of brain metastatic cells. Transcriptional expression of Fn14 was significantly activated in BRV5 cells (p=0.0031), similar to control HT29 cells (p=0.0122), suggesting TNFR2F12A dependence from TWEAK levels (Figure 2). In contrast, TWEAK decreased TNFR2F12A gene expression in 435-P cells with regard to controls (p=0.0246) and no differences were found in TNFR2F12A gene expression in 435-Br1 cells challenged with this cytokine. These results indicated that expression of TNFR2F12A was independent of TWEAK concentration in parental and in cells with low metastatic ability whereas brain metastasis aggressiveness was associated to TWEAK-Fn14 axis activation. In addition, we analyzed by flow cytometry Fn14 expression of BRV5 and HT29 cells stimulated with TWEAK. These experiments confirmed that Fn14 levels increased in TWEAK stimulated cells: six-fold in HT29 and three-fold in BRV5 cells in comparison to their respective controls. Taken together, this data confirms prior observation on the role of Fn14 previously associated with breast cancer to brain metastasis progression, where microenvironmental TWEAK-reactive astrocytes supported aggressiveness and angiogenesis [Martinez-Aranda et al., Oncotarget, 2015, 6, 44254-44273; Martinez-Aranda et al, Front Oncol., 2017, 7, 283] and suggested a role of TWEAK-Fn14 axis in signaling activation associated to brain metastasis progression.

It has been reported that transcriptional activation of Fn14 would be operated mainly by the NF-κB non-canonical pathway, through inducing the primary kinase NIK (MAP4K4) that phosphorylates IKKα [Vucid, Front Immunol., 2013, 4, 472]; thus we assessed the level of expression of NIK from 6 to 24 hours after cells were treated with TWEAK in a time-course RT-PCR (Figure 3). These results showed that the highest NIK expression occurred 6 hours after cell cultures were challenged with TWEAK in both, HT29 positive control cells and in the more aggressive brain metastatic BRV5 cells.

TWEAK independent cells, as 435-P and 435-Br1, showed similar NIK mRNA quantities, either with or without TWEAK, whereas the expression of NIK increased in TWEAK stimulated BRV5 cells after 6 hours of treatment, decreasing at 12 hours and partially recovered at 24 h. From these experiments we concluded that TWEAK-Fn14 pathway is mainly activated at 6 hours after treating cells, and thus we designed out experiments to check the inhibitory ability of the peptides at this specific time point.

The level of expression of probe genes on TWEAK-stimulated (T) BRV5 cells is overall higher than on non-stimulated (NT) cells. Peptides 1, 2, 3 and 4 displayed low overall expression across all genes in the TWEAK-Fn14 signaling pathway, as shown in Table 1.

**Table 1. Level of expression of probe genes in the absence or presence of peptides. The values represent the normalized level of expression (Zscores) taking as a reference the level of expression of housekeeping gene NARS2.**

| **Probe Gene** | **No Peptide** | **Peptide 1** | **Peptide 2** | **Peptide 3** | **Peptide 4** |
|---|---|---|---|---|---|
| TRAF2 | 2.29186659 | -0.54785027 | -0.40888041 | -0.57658919 | -0.43857745 |
| TNFRSF12A | 1.79997907 | -1.04756153 | -1.10855936 | -1.08194574 | -1.11204345 |
| MAP4K4 | 0.61954798 | -1.70176156 | -1.12531106 | -1.35943691 | -1.55513496 |
| NFkB2 | 1.40795522 | -1.50626609 | -1.20879820 | -1.15258257 | -1.32315046 |
| MAPK14 | 2.33192479 | -1.11066992 | -1.00585512 | -0.58531411 | -1.11241758 |
| JUN | -0.79378311 | -0.50852643 | -0.48826306 | -0.20615156 | -0.50948689 |
| IL6 | -1.55849043 | 0.19840981 | -0.53508236 | -0.97167045 | -0.04503358 |
| HSP90B1 | 0.87979442 | -1.10142657 | -1.30857990 | -0.46777542 | -0.92084596 |
| BIRC2 | 2.98026903 | -0.74681060 | -0.99630185 | -0.11502827 | -0.60950681 |

TWEAK was chosen as ligand, while TWEAK-Fn14 inhibitory peptides were chosen as analytes and passed across ligand-derivatized surface. Nitrilotriacetic acid-coated sensor chips (NTA chip) and a coupling approach [Kimple et al., Methods Mol Biol, 2010, 627, 91-100] that results in the capture of a His6-protein in a non-random orientation by the His6 epitope prior to direct free amine-based covalent coupling was used for histidine-tagged TWEAK immobilization.

Purified His6-TWEAK protein was successfully immobilized at a moderate density (∼7000 RUimmob) on the NTA sensor chip and the newly created surface was tested for functionality by conducting SPR binding assays using a monoclonal anti-TWEAK antibody and giving an affinity KD of 75 nM that agrees with the literature values for the cognate TWEAK-Fn14 pair [Day et al., Biochemistry, 2012, 51, 9124-9136]. After chip validation, peptides were tested. Figure 4 shows the set of binding sensorgrams obtained for various concentrations, ranging from 6.25 µM to 800 µM, of peptides 1-3, binding to His6-TWEAK covalently immobilized on the sensor chip surface.

For each titration, a zero-analyte surface as a control was subtracted from test sensorgram as well as non-specific binding to the blank flow cell without protein; and the chip surface was regenerated in order to remove any remaining analyte. The black curves show the experimental data, while the overlaid red lines represent a global fit of the entire data set to a 1: 1 Langmurian interaction model. The fitting process provided both kinetic association and dissociation rate constants (Ka and Kd), as well as equilibrium dissociation constant (KD) values indicated in Figure 4. Good fitting of experimental data to the calculated curves has been observed, suggesting the correctness of the used model. Furthermore, as expected for interactions involving low molecular weight peptides, the dissociation constants obtained were in the micromolar range with the peptide 2 (Figure 4B) presenting slightly higher affinity for TWEAK (i.e., KD of 170 µM).

These observations provide evidence that the TWEAK-Fn14 inhibitory peptides of the invention are able to bind TWEAK protein with reasonable affinities values in the 100-500 µM range.

## Claims

1. A peptide selected from the group consisting of TTDGSTGDDLQA (SEQ ID NO: 1), SWYDHMAGDSSY (SEQ ID NO: 2), SYTDVQDS (SEQ ID NO: 3) and DNGGGNTDIYSE (SEQ ID NO: 4) or a pharmaceutically acceptable salt thereof.

2. Peptide according to claim 1, wherein the peptide is TTDGSTGDDLQA (SEQ ID NO: 1).

3. Peptide according to claim 1, wherein the peptide is SWYDHMAGDSSY (SEQ ID NO: 2).

4. Peptide according to claim 1, wherein the peptide is SYTDVQDS (SEQ ID NO: 3).

5. Peptide according to claim 1, wherein the peptide is DNGGGNTDIYSE (SEQ ID NO: 4).

6. Composition comprising a peptide according to any one of claims 1 to 5 and a pharmaceutically acceptable excipient.

7. Peptide according to any one of claim 1 to 5 or composition according to claim 6, for use in medicine.

8. Peptide according to any one of claim 1 to 5 or composition according to claim 6, for use in the treatment and/or prevention of an Fn-14 positive solid cancer.

9. Peptide or composition for use according to claim 8, wherein the solid cancer is selected from the group consisting of brain cancer, breast cancer, lung cancer, melanoma, liver cancer, colorectal cancer, esophageal cancer, bladder cancer, pancreatic cancer, ovarian cancer, prostate cancer and neuroblastoma.

10. Peptide or composition for use according to claim 9, wherein the solid cancer is brain cancer.

11. Peptide or composition for use according to claim 10, wherein the brain cancer is metastatic brain cancer.

12. Peptide or composition for use according to claim 11, wherein the metastatic brain cancer is brain metastasis of an Fn-14 positive cancer selected from the group consisting of breast cancer, lung cancer, melanoma, colon cancer, and kidney cancer.

13. Peptide or composition for use according to claim 12, wherein the metastatic brain cancer is brain metastasis of breast cancer.

14. Peptide or composition for use according to claim 9, wherein the brain cancer is a glioblastoma.

15. Peptide according to any one of claim 1 to 5 or composition according to claim 6, for use in the treatment and/or prevention of cancer-induced cachexia.
